# EUROPÄISCHE PATENTANMELDUNG

(11) **EP 1 192 989 A1**
(43) Veröffentlichungstag der Anmeldung: **03.04.2002**
(21) Anmeldenummer: 00121075.6
(22) Anmeldetag: 28.09.2000
(51) Int. Cl.: B01J 23/42, B01J 23/44, C07C 209/36, C09C 1/50

(54) **Katalysator zur Hydrierung aromatischer Nitroverbindungen**

(71) Anmelder: Degussa AG, 40474 Düsseldorf (DE)
(72) Erfinder: Albers, Peter, Dr., 63454 Hanau (DE); Auer, Emanuel, Dr., 60528 Frankfurt a. Main (DE); Gross, Michael, 60385 Frankfurt a. Main (DE); Krauter, Jürgen, Dr., 63768 Hösbach (DE); Packruhn, Uwe, 60389 Frankfurt a. Main (DE)

(57) **Zusammenfassung**

Hydrierkatalysator, enthaltend als Kohlenstoffträger einen Ruß mit einem H-Gehalt von > 4000 ppm und als katalytisch aktive Komponente Palladium und/oder Platin oder bi- und/oder miltimetallisch dotiertes oder legiertes Palladium und/oder Platin, wird hergestellt, indem man zu einer Suspension des Rußes mit einem H-Gehalt > 4000 ppm Metallsalzlösungen zugibt, durch Verwendung einer basischen Verbindug die Metallsalzlösungen hydrolysiert und die vollständige Abscheidung des Metalls durch Reduktion mit einem Reduktionsmittel durchgeführt.
Der Hydrierkatalysator kann zur Hydrierung von Nitroaromaten eingesetzt werden.

## Beschreibung

Die vorliegende Erfindung betrifft einen pulverförmigen Hydrierkatalysator, ein Verfahren zu seiner Herstellung und seine Verwendung in der katalytischen Suspensionshydrierung aromatischer Nitroverbindungen.

Aromatische Amine stellen heute zentrale Bausteine in der Herstellung von Polymeren, gummitechnischen Erzeugnissen, Agro- und Pharmachemikalien dar. Insbesondere Anilin und Toluoldiamin sind wichtige Zwischenprodukte in der Synthese von Iso- und/oder Diisocyanaten, die als Monomere für die Herstellung von Polyurethanen in Form verschiedener Werkstoffe (Schäume, Elastomere) verwendet werden.

Des weiteren finden MDA (Methylendianilin), ein Kondensationsprodukt aus 2 mol Anilin mit Formaldehyd, sowie das stereoselektiv ringhydrierte Folgeprodukt Bis-para-amino-cyclohexylmethan (PACM) verbreitet Anwendung in der Polymerindustrie.

Für die Herstellung der aromatischen Amine durch katalytische Hydrierung der entsprechenden Nitroverbindung werden heute im industriellen Maßstab eine Reihe unterschiedlicher Verfahren und/oder Katalysatoren angewendet. Neben der Gasphasenhydrierung von Nitrobenzol, die insbesondere zur Herstellung von Anilin eingesetzt wird, gibt es eine Reihe von Verfahren, die in Flüssigphase arbeiten. Dabei finden sowohl SiO₂-geträgerte Unedelmetallkatalysatoren als auch aktivierte Metallkatalysatoren von Raney® -Typ Verwendung.

Der Einsatz von edelmetallhaltigen Katalysatoren für die katalytische Hydrierung von aromatischen Nitroverbindungen in flüssiger Phase ist seit langem bekannt (G. C. Bond, P. B. Walls, Advan. Catal. Relat. Subj. 15, 1964, 92).

Obwohl Palladium-Katalysatoren für die katalytische Hydrierung sowohl von Nitrobenzol (NB) zu Anilin als auch von Dinitrotoluol (DNT) zu Toluoldiamin (TDA) in der industriellen Anwendung verbreitet sind, ist eines der zentralen Probleme die Deaktivierung des Katalysators durch die Bildung unerwünschter Nebenprodukte, wie zum Beispiel ringhydrierter Derivate oder Dimerisierungs -und/oder Oligomerisierungsprodukte von partiell hydrierten Zwischenprodukten der Reaktion, die in der Literatur unter dem Begriff "Teerbildung" zusammengefasst sind.

Bekannt ist eine Reihe von Publikationen, die sich mit Methoden beschäftigt, durch Auswahl eines geeigneten Trägermaterials und Modifikation der Palladiumkatalysatoren mit Eisen oder anderen Metallen die Selektivität in der Flüssigphasen-Hydrierung von aromatischen Nitroverbindungen zu erhöhen und gleichzeitig die Ausbeute an Amin zu verbessern.

Bekannt ist die Verwendung von modifizierten Palladiumkatalysatoren (EP 002 308 B1). Dabei wird durch den Einsatz eines hochoberflächigen Aktivkohleträgers eine sehr hohe Dispersion des Palladiums auf der Trägeroberfläche erreicht. Dies führt zu einer Verbesserung der Aktivität in der katalytischen Hydrierung von Dinitrotoluol, die in Methanol oder anderen geeigneten Solventien durchgeführt wird.

Insbesondere die sehr gute Edelmetalldispersion auf dem Träger trägt zu einer selektiv verlaufenden Hydrierreaktion bei, da die hohe Wärmetönung der Nitrogruppenhydrierung zur Bildung unerwünschter Nebenprodukte ("Überhydrierung") führen kann. Deshalb ist eine hohe Edelmetalldispersion auf dem Träger notwendig, um die bei der Hydrierung auftretende Exothermie am Reaktionszentrum sofort auf das Trägermaterial zu dissipieren (Wärmeabfuhr).

Weiterhin ist bekannt, einen eisenmodifizierten Palladiumkatalysator auf einem hydrophoben Rußträger ("oleophiler Ruß") herzustellen (US 3 127 356). Insbesondere die Verwendung eines sehr feinteiligen Rußträgers wie Acetylenruß und die Dotierung des Palladiums mit Elementen, wie Eisen und/oder Platin, führt bei dem bekannten Verfahren zu einer deutlichen Verbesserung der Aktivitätsrate (Umsatz Dinitrotoluol bezogen auf eingesetztes Metall) sowie zu einer Erhöhung der Selektivität.

Die Auswahl eines geeigneten Rußträgers besitzt den Vorteil, daß durch die gegenüber Aktivkohle hohe Wärmeleitfähigkeit des Trägermaterials eine schnelle Abführung der Exothermie möglich ist.

Allerdings besitzt der in US 3 127 356 beschriebene oleophile Acetylenruß den Nachteil, daß eine hochdisperse Abscheidung der Metalle Palladium, Platin und Eisen in wässriger Suspension wegen der hydrophoben Oberfläche des Rußes nicht optimal möglich ist. Aus diesem Grund weisen Katalysatoren, die auf einem Acetylenruß (zum Beispiel Shawinigan Black der Firma Chevron) gemäß Beispiel 1 der US 3 127 356 hergestellt werden, nur eine begrenzte Aktivität und Selektivität in der katalytischen Hydrierung von Dinitrotoluol auf.

Aufgabe der vorliegenden Erfindung ist es, einen rußgeträgerten Hydrierkatalysator herzustellen, der eine höhere Edelmetalldispersion besitzt und aktiver und selektiver ist als die bekannten Katalysatoren.

Gegenstand der Erfindung ist ein Hydrierkatalysator, enthaltend als Kohlenstoffträger einen Ruß mit einem H-Gehalt von > 4000 ppm, vorzugsweise > 4200 ppm, besonders bevorzugt > 4400 ppm, bestimmt durch CHN-Analytik, und als katalytisch aktive Komponente Palladium und/oder Platin oder bi- und/oder multimetallisch dotiertes oder legiertes Palladium und/oder Platin enthält.

Bi- und/oder multimetallisch dotiertes oder legiertes Palladium und/oder Platin können durch Dotierungen des Palladiums und/oder Platins beziehungsweise Legierungen von Palladium und/oder Platin mit den Elementen Fe, V, Rh, Sn, Ru oder Kombinationen davon enthalten werden.

Das Verhältnis von CTAB-Oberfläche (Cetylammoniumbromid) zu BET-Oberfläche kann beim erfindungsgemäßen Ruß 0,9 - 1,1 betragen.

Ein CTAB/BET-Oberflächenverhältnis des Rußes von nahe 1 erlaubt die hochdisperse Abscheidung aktiver Metallkomponenten auf dem Träger, ohne daß sich Edelmetallkristallite in den Poren des Rußträgers abscheiden, und ihre spezifische Metalloberfläche wegen Massentransportlimitierung für Substratmoleküle nicht mehr zugänglich ist.

Als Ruß mit einem H-Gehalt von größer 4000 ppm, bestimmt durch CHN-Analytik, kann ein Ruß mit einem H-Gehalt von größer 4000 ppm und einem Peaktintegralverhältnis, bestimmt durch inelastische Neutronenstreuung (INS), von nichtkonjugierten H-Atomen (1250-2000 cm⁻¹) zu aromatischen und graphitischen H-Atomen (1000-1250 cm⁻¹) und 750-1000 cm⁻¹) zu kleiner 1,22, vorzugsweise kleiner 1,20, eingesetzt werden.

Die Herstellung des Furnacerußes kann in einem Rußreaktor, welcher längs der Reaktorachse eine Verbrennungszone, eine Reaktionszone und eine Abbruchzone enthält, erfolgen. In der Verbrennungszone wird durch vollständiges Verbrennen eines Brennstoffes in einem Sauerstoff enthaltenden Gas ein Strom heißen Abgases erzeugt. In der Reaktionszone werden anschließend Rußrohstoffe in das heiße Abgas eingemischt. Das Abstoppen der Rußbildung erfolgt in der Abbruchzone durch Einsprühen von Wasser, wobei ein flüssiger und gasförmiger Rußrohstoff an derselben Stelle eingedüst werden.

Der flüssige Rußrohstoff kann durch Druck, Dampf, Pressluft oder den gasförmigen Rußrohstoff zerstäubt werden.

Flüssige Kohlenwasserstoffe verbrennen langsamer als gasförmige, da sie zuerst in die Gasform überführt, das heißt, verdampft werden müssen. Hierdurch befinden sich im Ruß Anteile, die aus dem Gas gebildet werden und solche die aus der Flüssigkeit gebildet werden.

Als Maßzahl zur Kennzeichnung des Luftüberschusses wird häufig der sogenannte K-Faktor verwendet. Es handelt sich bei dem K-Faktor um das Verhältnis der für eine stöchiometrische Verbrennung des Brennstoffes benötigten Luftmenge zu der tatsächlich der Verbrennung zugeführten Luftmenge. Ein K-Faktor von 1 bedeutet also eine stöchiometrische Verbrennung. Bei Luftüberschuß ist der K-Faktor kleiner 1. Dabei können wie bei bekannten Rußen K-Faktoren zwischen 0,3 und 0,9 angewendet werden. Bevorzugt wird mit K-Faktoren zwischen 0,6 und 0,7 gearbeitet.

Als flüssiger Rußrohstoff können flüssige aliphatische oder aromatische, gesättigte oder ungesättigte Kohlenwasserstoffe oder Mischungen hiervon, Destillate aus dem Steinkohlenteer oder Rückstandsöle, die beim katalytischen Cracken von Erdölfraktionen beziehungsweise bei der Olefinherstellung durch Cracken von Naphtha oder Gasöl entstehen, eingesetzt werden.

Als gasförmiger Rußrohstoff können gasförmige aliphatische, gesättigte oder ungesättigte Kohlenwasserstoffe, Mischungen hiervon oder Erdgas eingesetzt werden.

Das beschriebene Verfahren ist nicht auf eine bestimmte Reaktorgeometrie beschränkt. Es kann vielmehr auf verschiedene Reaktortypen und Reaktorgrößen angepasst werden. Als Rußrohstoffzerstäuber können sowohl reine Druckzerstäuber (Einstoffzerstäuber) als auch Zweistoffzerstäuber mit innerer oder äußerer Mischung eingesetzt werden, wobei als Zerstäubungsmedium der gasförmige Rußrohstoff verwendet werden kann. Die vorstehend beschriebene Kombination eines flüssigen mit einem gasförmigen Rußrohstoff kann also zum Beispiel durch Verwendung des gasförmigen Rußrohstoffs als Zerstäubungsmedium für den flüssigen Rußrohstoff realisiert werden.

Bevorzugt können zur Zerstäubung von flüssigem Rußrohstoff Zweistoffzerstäuber eingesetzt werden. Während bei Einstoffzerstäubern eine Änderung des Durchsatzes auch zu einer Änderung der Tröpfchengröße führt, kann die Tröpfchengröße bei Zweistoffzerstäubern weitgehend unabhängig vom Durchsatz beeinflusst werden.

Die CTAB-Oberfläche kann von 20 bis 400 m²/g, vorzugsweise von 20 bis 150 m²/g, betragen. Die DBP-Zahl kann von 40 bis 200 ml/100 g, vorzugsweise 100 bis 180 ml/100 g, betragen.

Als Ruß mit einem Wasserstoffgehalt > 4000 ppm, bestimmt durch CHN-Analytik, kann weiterhin ein Ruß eingesetzt werden, der aus der DE 19521565 bekannt ist.

Die Ruße können unbehandelt oder nachbehandelt eingesetzt werden. Der Ruß kann undotiert oder mit Fremdatomen dotiert sein. Fremdatome können Si, Zr, Sb, V, Fe, Mg oder Ti sein.

Der sehr hohe Wasserstoffgehalt ist ein Hinweis auf eine starke Störung des Kohlenstoffgitters durch erhöhte Zahl an Kanten der im Vergleich zum Acetylenruß (zum Beispiel Shawinigan Black) kleineren C-Kristallite. Der Wasserstoffanteil kann mittels Neutronenbeugung zweifelsfrei nachgewiesen werden und deutet auf die Existenz sp³-hybridisierter C-Atome, sogenannte Störstellen im Kristallitgitter an, auf denen Palladium, Eisen oder Platin bevorzugt abgeschieden werden können.

Für eine optimale Funktion der erfindungsgemäßen Hydrierkatalysatoren kann die Beladung zwischen 0,05 und 80 Gew. % Palladium und/oder Platin, bevorzugt zwischen 0,5 und 10 Gew. %, bezogen auf das Gesamtgewicht des Katalysators, betragen.

Die Atomverhältnisse zwischen Palladium und/oder Platin und den gegebenenfalls mehreren anderen Dotierungs- und/oder Legierungselementen können zwischen 200 : 1 und 1 : 200 bevorzugt jedoch zwischen 100 : 1 und 1 : 100 liegen.

Im Falle von tri- oder multimetallischen Hydrierkatalysatoren kann das Atomverhältnis der weiteren Legierungskomponenten untereinander in den Grenzen zwischen 100 : 0 und 0 : 100 variiert werden. Besonders vorteilhaft sind jedoch Atomverhältnisse in den Grenzen 50 : 1 und 1 : 50.

Ein weiterer Gegenstand der Erfindung ist ein Verfahren zur Herstellung des erfindungsgemäßen Hydrierkatalysators, welches dadurch gekennzeichnet ist, daß zu einer Suspension eines Rußes mit einem H-Gehalt > 4000 ppm, Edelmetallsalzlösung und gegebenenfalls Salzlösungen der Dotierungs- beziehungsweise Legierungselemente gleichzeitig, nacheinander oder in einem zweistufigen Verfahren nach vorheriger Herstellung eines Edelmetallvorkatalysators zugegeben wird, durch Verwendung einer basischen Verbindung die (Edel-) Metallsalzlösungen hydrolysiert als Hydroxide auf dem Träger ausgefällt werden und die vollständige Abscheidung des Edelmetalls beziehungsweise der anderen Metalle durch Reduktion mit einem Reduktionsmittel durchgeführt wird. Die Reduktion kann bei einer Temperatur von 0 bis 100°C durchgeführt werden.

Gegebenenfalls kann die Reduktion mit Wasserstoffgas in Flüssigphase oder am getrockneten Katalysator erfolgen. Die Reihenfolge des Zusammenfügens von Trägermaterial, Wasser, Metallsalzlösungen und wasserlöslichen Reduktionsmitteln kann auch variiert werden. Als geeignete nasschemische Reduktionsmittel können beispielsweise Formaldehyd, Hydrazin oder Natriumborhydrid verwendet werden.

Die Verwendung eines Reduktionsmittels ist optional, d. h. der erfindungsgemäße Katalysator kann auch nach Hydrolyse der (Edel-) Metallsalzlösungen ohne weitere Reduktion durch Filtration von der Reaktionsmischung abgetrennt werden.

Nach der Abtrennung des Katalysators durch Filtration kann sich ein Trocknungsschritt anschließen. Es kann außerdem im Anschluss an die naßchemische Präparation eine Temperaturbehandlung unter Inertgas oder reduzierender Atmosphäre bei Temperaturen zwischen 0°C und 1000°C, vorzugsweise zwischen 100°C und 700°C durchgeführt werden.

Der erfindungsgemäße Hydrierkatalysator kann zur Hydrierung von Nitroaromaten eingesetzt werden. Der erfindungsgemäße Katalysator kann insbesondere zur Hydrierung von Nitrobenzol zu Anilin und von Dinitrotoluol zu Toluoldiamin eingesetzt werden.

Die katalytische Hydrierung der Nitroverbindungen kann in Flüssigphase als kontinuierlich oder diskontinuierlich betriebener Prozess bei Drücken zwischen 1 und 100 bar und Temperaturen zwischen 0 °C und 250 °C in Gegenwart des erfindungsgemäßen Katalysators durchgeführt werden.

Die katalytische Hydrierung von Nitrobenzol oder Dinitrotoluol in Gegenwart des erfindungsgemäßen Katalysators kann im diskontinuierlichen oder kontinuierlich betriebenen Rührreaktor in Gegenwart eines Lösungsmittels, wie zum Beispiel Methanol oder Toluol, erfolgen. Sie kann auch in einem Gemisch aus Anilin/Wasser oder Toluoldiamin/Wasser insbesondere bei kontinuierlichen Verfahren erfolgen.

Die Hydrierung von Dinitrotoluol zu Toluoldiamin kann bei Temperaturen zwischen 70 und 200°C, vorzugsweise 90 und 150°C, und Drücken zwischen 1 und 100 bar, vorzugsweise 25 bis 50 bar durchgeführt werden. Wird die Hydrierung kontinuierlich betrieben, so muß die Menge an umgesetztem Edukt durch Nachdosieren ersetzt und das Produkt / Wassergemisch aus dem Reaktor entfernt werden.

Die erfindungsgemäße Hydrierung zu Toluoldiamin in Gegenwart des erfindungsgemäßen Katalysators zeichnet sich vorteilhafterweise vor allem durch eine niedrige Nebenproduktbildung und hohe Ausbeuten an Toluoldiamin aus. Die unerwünschte Bildung ringhydrierter Derivate und unvollständig hydrierter Intermediate wird nicht beobachtet. Auch die Di- und Oligomerisierung unterschiedlicher Reaktionszwischenstufen ("Teerbildung") ist deutlich geringer. Die Ausbeute an Toluoldiamin liegt in allen Fällen über denen, die unter Verwendung von bekannten rußgeträgerten Palladium- oder modifizierter Palladiumkatalysatoren erzielt werden konnten.

Die erfindungsgemäßen Katalysatoren zeichnen sich durch eine hohe Dispersion der auf dem Träger abgeschiedenen Metallpartikel sowie eine höhere Aktivität und Selektivität in der katalytischen Hydrierung von nitroaromatischen Verbindungen (zum Beispiel Anilin, Dinitrotoluol) aus.

### Beispiele

Gemäß den folgenden Beispielen und Vergleichsbeispielen werden erfindungsgemäße Hydrierkatalysatoren und Vergleichskatalysatoren hergestellt und bezüglich ihrer katalytischen Eigenschaften in der Hydrierung von Nitroaromaten miteinander verglichen.

Als Trägermaterial für den Erfindungsgemäßen Elektrokatalysator wird der Ruß B2 der Firma Degussa-Hüls eingesetzt, für die Vergleichskatalysatoren der Acetylenruß Shawinigan Black der Firma Chevron.

### Herstellung des Rußes:

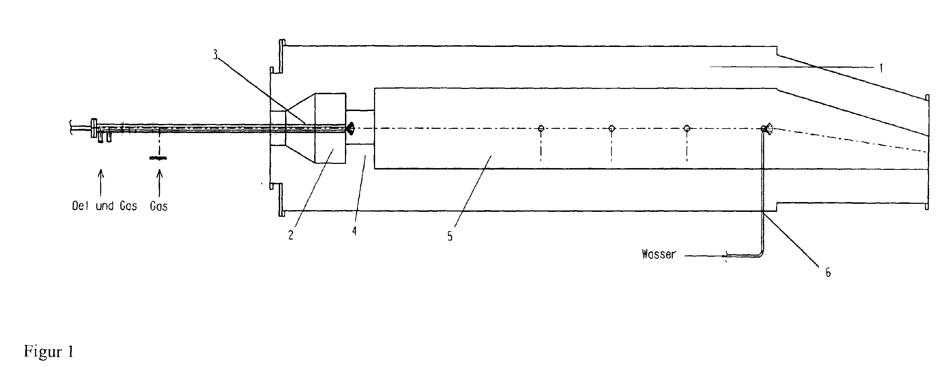

Der Ruß B2 wird in dem in Figur 1 dargestellten Rußreaktor 1, durch Eindüsen des flüssigen und gasförmigen Rußrohstoffes an derselben Stelle, hergestellt. Dieser Rußreaktor 1 besitzt eine Brennkammer 2. Das Öl und Gas wird über die axiale Lanze 3 in die Brennkammer eingeführt. Die Lanze kann zur Optimierung der Rußbildung in axialer Richtung verschoben werden.

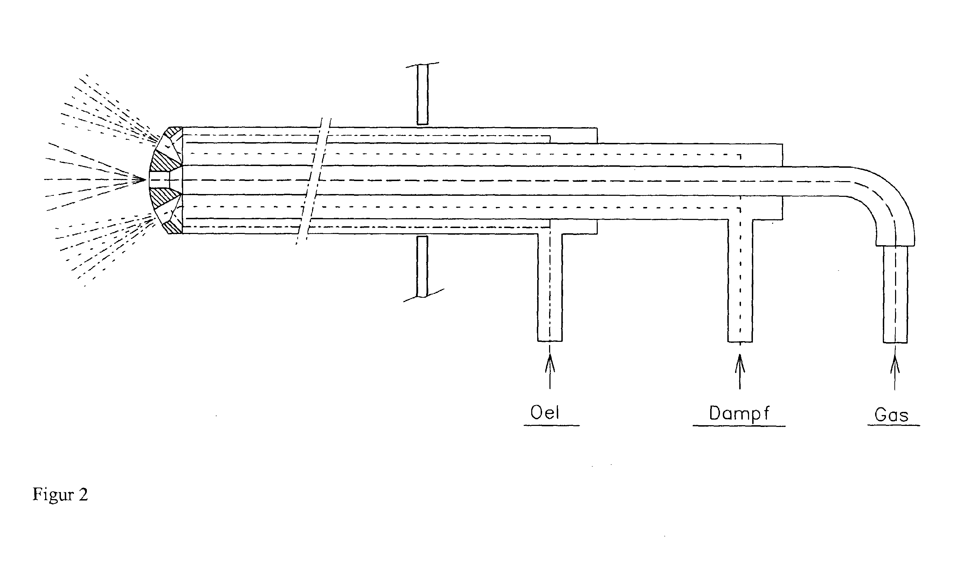

Die Brennkammer läuft auf die Engstelle 4 zu. Nach Durchqueren der Engstelle expandiert das Reaktionsgasgemisch in die Reaktionskammer 5. Die Lanze weist an ihrem Kopf geeignete Sprühdosen auf (Figur 2) Die für das Verfahren wichtige Verbrennungszone, Reaktionszone und Abbruchzone können nicht scharf voneinander getrennt werden. Ihre axiale Ausdehnung hängt von der jeweiligen Positionierung der Lanzen und der Quenchwasser-Lanze 6 ab.

Die Abmessungen des verwendeten Reaktors sind der folgenden Aufstellung zu entnehmen:

| | |
|---|---|
| Größter Durchmesser der Brennkammer: | 696 mm |
| Länge der Brennkammer bis Engstelle: | 630 mm |
| Durchmesser der Engstelle: | 140 mm |
| Durchmesser der Reaktionskammer: | 802 mm |
| Position der Öllanzen | + 160 mm |
| Position der Quenchwasser-Lanzen ¹⁾ | 2060mm |

| | |
|---|---|
| ¹⁾ gemessen vom Nullpunkt (Beginn der Engstelle) | |

Die Reaktorparameter für die Herstellung des erfindungsgemäßen Rußes sind in der folgenden Tabelle aufgeführt.

| **Reaktorparameter** | **Ruß** | |
|---|---|---|
| **Parameter** | **Einheit** | **B2** |
| Verbrennungsluft | Nm³/h | 1500 |
| Temperatur der Verbrennungsluft | °C | 550 |
| Σ Erdgas | Nm³/h | 156 |
| k-Faktor (gesamt) | | 0,70 |
| Rußöl, axial | kg/h | 670 |
| Rußöl-Stellung | Mm | + 16 |
| Zerstäuberdampf | kg/h | 100 |
| Additiv (K₂CO₃-Lösung) | 1/h x g/l | 5,5 x 3,0 |
| Additiv Position | | axial |
| Reaktorausgang | °C | |
| Quenchposition | mm | 749 |
| | | 9/8810 |

### Charakterisierung der Trägermaterialien:

Die Wasserstoffgehalte der beiden Ruße werden sowohl durch CHN-Elementanalytik als auch mittels Neutronenbeugung bestimmt. Die Methode der inelastischen Neutronenstreuung (INS) ist in der Literatur beschrieben (P. Albers, G. Prescher, K. Seibold, D. K. Ross and F. Fillaux, Inelastic Neutron Scattering Study Of Proton Dynamics In Carbon Blacks, Carbon 34 (1996) 903 und P. Albers, K. Seibold, G. Prescher, B. Freund, S.F. Parker, J. Tomkinson, D.K. Ross, F. Fillaux, Neutron Spectroscopic Investigations On Different Grades Of Midified Furnace Blacks And Gas Blacks,Carbon 34 (1999) 437).

Die Methode INS (beziehungsweise IINS - Inelastische, inkohärente Neutronenstreuung) bietet einige recht einmalige Vorteile für die noch intensivere Charakterisierung von Rußen und Aktivkohlen.

Ergänzend zur bewährten elementanalytischen Quantifizierung des H- Gehaltes, erlaubt es die INS-Methode den zum Teil recht geringen Wasserstoffanteil in graphitisierten Rußen (ca. 100-205ppm), Rußen (ca. 2000-4000 ppm in Furnacerußen) und in Aktivkohlen (ca. 5000-12000 ppm in typischen Katalysatorträgern) detaillierter hinsichtlich seiner Bindungszustände aufzuschlüsseln.

Zu Vergleichszwecken sind in der folgenden Tabelle die mittels CHN-Analytik (Leco-404-Analysator mit Wärmeleitfähigkeitsdetektor) bestimmten Werte des Gesamtwasserstoffgehaltes der Ruße aufgeführt. Zudem sind die auf das Probengewicht normierten Spektrenintegrale angegeben, die folgendermaßen bestimmt werden: Integration des Bereiches eines INS-Spektrums von 500-3600 cm⁻¹. Hierdurch wird die Graphitschwingungsbande der Kohlenstoffmatrix bei ca. 110 cm⁻¹ ausgeblendet.

| **Ruß** | **H-Gehalt [ppm] durch CHN- Elementanalytik** | **H-Gehalt [Integral/Probeng ewicht] durch INS** |
|---|---|---|
| B2 | 4580 ± 300 | 69.1 |
| Shawinigan Black Acetylen Ruß | 800 | 46.5 |

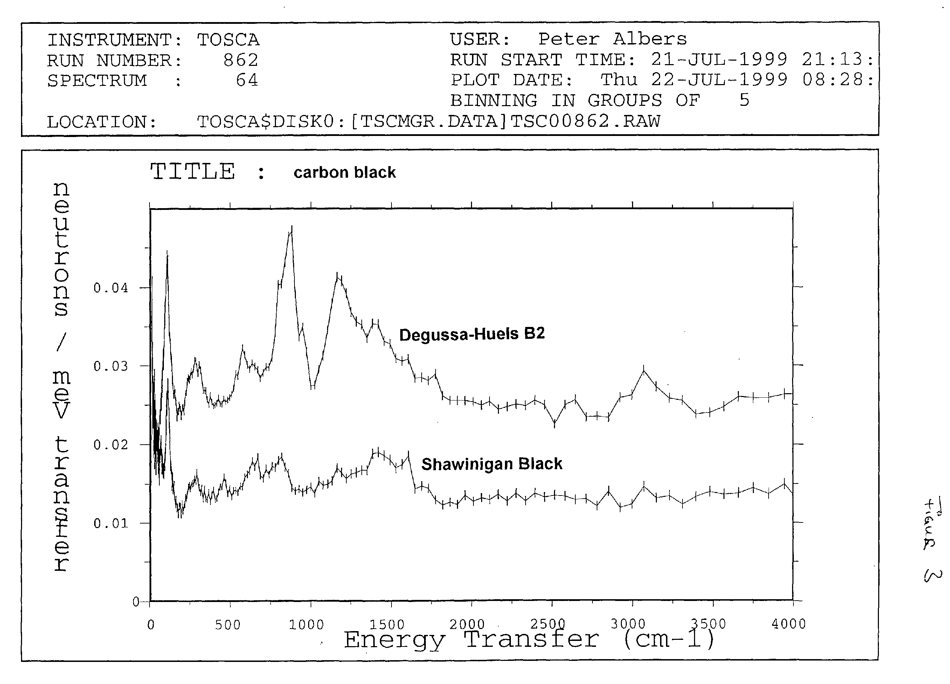

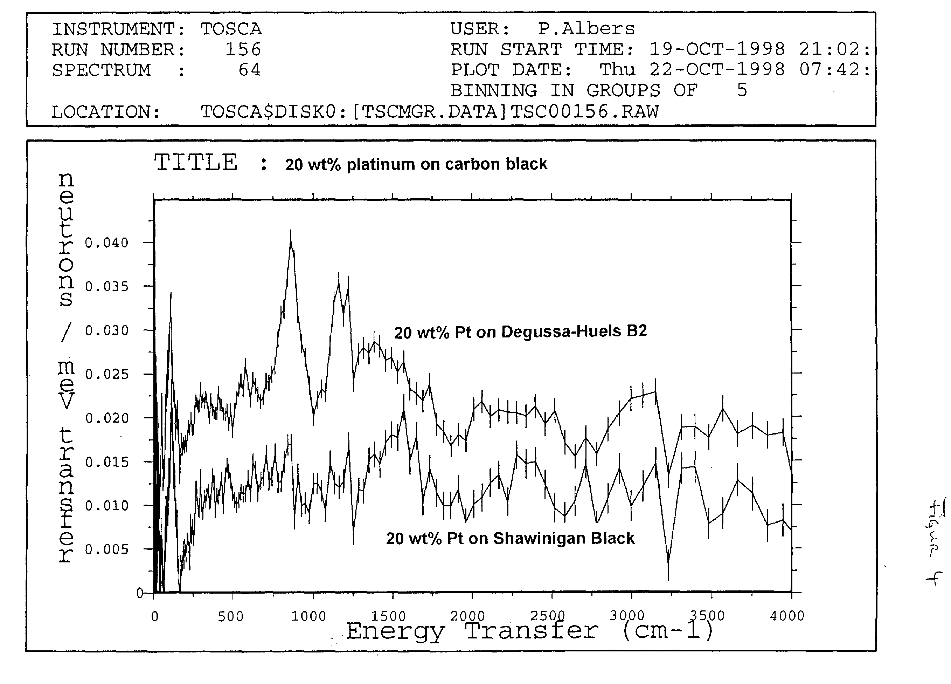

In Figur 3 sind exemplarisch die Spektren der inelastischen Neutronenstreuung für Shawinigan Black und Ruß B2 dargestellt. In Figur 4 finden sich die INS-Spektren zweier Platinkatalysatoren auf Shawinigan Black beziehungsweise dem Degussa-Huels Ruß B2 (erfindungsgemäße Katalysator). Man erkennt deutlich die Unterschiede im Schwingungsbereich für die Cₛₚ₃-H Schwingung der beiden Ruße.
Für den Materialvergleich der beiden Ruße sind - neben der Graphitschwingungsbande bei 112 cm⁻¹ - folgende Bereiche von Bedeutung:
1. der Bereich von 750 - 1000 cm⁻¹ (d.h. bis zur scharfen Separation bei 1000 cm⁻¹); er entspricht den "out of plane" C-H-Deformationsschwingungsbanden an den Abbruchkanten der Netzebenen der graphitischen Rußbausteine
2. der Bereich von 1000 - 1250 cm⁻¹; er entspricht den "in plane" C-H-Deformationsschwingungsbanden
3. der Bereich 1250 - 2000 cm⁻¹; er entspricht den C-H-Deformationsschwingungen nicht-konjugierter Bestandteile.

In der folgenden Tabelle sind neben den Bereichsintegralen der genannten Spektrensegmente versuchsweise auch einige Quotienten dieser Werte angegeben; zudem zeigt der Vergleich mit dem Gesamt-Wasserstoffgehalt eine recht zufriedenstellende Korrelation zwischen diesen INS-Werten und den Ergebnissen der CHN-Analytik:

| | A | B | C | C/A | C/(A+B) | H-Gehalt |
|---|---|---|---|---|---|---|
| | 750-1000 cm⁻¹ ± 1 | 1000-1250 cm⁻¹ ± 1 | 1250-2000 cm⁻¹ ± 3 | | | ppm |
| Shawinigan | 24 | 24 | 76 | 3.2 | 1.58 | 800 |
| Degussa-Hüls B2 | 107 | 99 | 241 | 2.25 | 1.17 | 4580 |

Ergebnisse der Spektrenintegrationen: Vergleich der Bereichsintegrale mit dem Gesamtwasserstoffgehalt gemäß CHN-Analytik.

Durch den deutlich höheren Wasserstoffgehalt ist der erfindungsgemäße Ruß B2 gegenüber den Shawinigan Black deutlich hydrophiler, was der hochdispersen Abscheidung des Edelmetalls entgegenkommt. Andere Eigenschaften der beiden Ruße, z.B. das Oberflächenverhältnis der spezifischen Gesamtoberfläche (bestimmt durch BET) und der CTAB-Oberfläche (bestimmt durch Cetylammoniumbromid-Adsorption gemäß DIN 66132) sind sehr ähnlich.

| **Ruß** | **CTAB Oberfläche [m**^{**2**}**/g]** | **BET Oberfläche [m**^{**2**}**/g]** | **BET : CTAB Oberflächen-verhältnis** |
|---|---|---|---|
| B2 | 40 | 40 | 1 |
| Shawinigan Black | 80 | 80 | 1 |

### Beispiele:

### 1. Palladium auf Shawinigan Black (Vergleichsbeispiel)

Es werden 23,6 g Shawinigan Black Ruß in deionisiertem Wasser suspendiert und der pH-Wert mit Natriumcarbonatlösung alkalisch gestellt (pH = 10.0). Zu dieser Suspension werden 0,44 g Palladium(II)-chloridlösung (20%ig) zugegeben. Nach dem Aufheizen auf 90°C wird ein pH-Wert von 6.5 eingestellt. Man rührt nach und filtriert den Katalysator ab. Der fertige Katalysator enthält 1,75 Gew.-% Palladium.

### 2. Palladium auf erfindungsgemäßem Ruß B2

In Analogie zu Beispiel 1 wird unter Verwendung des Degussa-Hüls Rußes B2 ein Palladiumkatalysator hergestellt. Die Metallbeladung auf dem Träger ist ebenfalls 1,75 Gew.-%.

### 3. Pd Katalysator auf Shawinigan Black gemäß US 3 127 356

Gemäß Beispiel 1 der US 3 127 356 wird ein monometallischer Pd-Katalysator auf Shawinigan Black hergestellt. Dabei wird in Abwandlung der Herstellvorschrift lediglich Palladium(II)chlorid als Metallsalz, nicht jedoch eine Eisenverbindung zur Herstellung verwendet.

### 4. Palladium/Eisen-Katalysator auf Shawinigan Black

Es werden 23,6 g Shawinigan Black Ruß in deionisiertem Wasser suspendiert und der pH-Wert mit Natriumcarbonat alkalisch gestellt (pH = 10.0). Zu dieser Suspension werden 1,05 g Eisen(III)chlorid in 100 ml deionisiertem Wasser und 0,44 g Palladium(II)chlorid (20%ig) zugegeben. Nach Aufheizen auf 90 °C wird ein pH von 6.5 eingestellt. Man rührt nach und filtriert den Katalysator ab. Der fertige Katalysator enthält 1,75 Gew.-% Palladium und 4.2 Gew.-% Eisen.

### 5. Pd/Fe auf Degussa-Hüls Ruß B2 (erfindungsgemäß)

In Analogie zu Beispiel 4 wird unter Verwendung des Degussa-Hüls Rußes B2 ein Pd/Fe-Katalysator mit einer Beladung von 1.75 Gew.-% Pd und 4.2 Gew.-% Fe hergestellt.

### 6. Pd/Fe Katalysator auf Shawinigan Black nach US 3 127 356

Gemäß Beispiel 3 der US 3 127 356 wird ein bimetallischer Pd/Fe-Katalysator auf Shawinigan Black hergestellt.

### 7. Pt auf Shawinigan Black

Es werden 24,75 g Shawinigan Black Ruß in deionisiertem Wasser suspendiert und der pH-Wert mit Natriumhydrogencarbonat alkalisch gestellt (pH = 8.0). Nach Aufheizen auf 90 °C werden zu dieser Suspension 1,00 g Hexachloroplatin(IV)säure (25%ig) zugegeben. Der pH-Wert wird erneut alkalisch gestellt (pH > 8.0) und bei 90 °C mit Formaldehydlösung (37%ig) reduziert. Man rührt nach und filtriert den Katalysator ab. Der fertige Katalysator enthält 1 Gew.-% Platin.

### 8. Pt auf Degussa-Hüls Ruß B2 (erfindungsgemäß)

In Analogie zu Beispiel 7 wird unter Verwendung des Degussa-Hüls Rußes B2 ein Platinkatalysator mit einer Edelmetallbeladung von 1 Gew.-% hergestellt.

### Charakterisierung der Katalysatoren

Die hergestellten Hydrierkatalysatoren wurden bezüglich ihrer Edelmetalldispersion über CO-Cemisorption und ihrer Aktivität im Nitrobenzolniederdrucktest charakterisiert.

| **Katalysator / Edelmetallgehalt** | **CO-Chemisorption [ml/g]** | **Dispersion [%]** | **Nitrobenzol Aktivität [ml/g min]** |
|---|---|---|---|
| Beispiel **1** 1,75% Pd | 0,98 | 26,4 | 920 |
| Beispiel **2** 1,75% Pd | 1,05 | 29,0 | 1000 |
| Beispiel **3** 1,75% Pd | 0,49 | 13,2 | 650 |
| Beispiel **4** 1,75% Pd 4,2% Fe | n.b.* | n.b.* | 60 |
| Beispiel **5** 1,75% Pd 4,2% Fe | n.b.* | n.b.* | 80 |
| Beispiel **6** 1,75% Pd 4,2% Fe | n.b.* | n.b.* | 20 |
| Beispiel **7** 1 % Pt | 0,54 | 47,0 | 300 |
| Beispiel **8** 1 % Pt | 0,87 | 76,0 | 650 |

| | | | |
|---|---|---|---|
| n.b. * bei bimetallischen Pd-Fe Katalysatoren können sich bei der CO-Chemisorptionsmessung hochtoxische Eisencarbonyle bilden, die die Messung verfälschen. Deshalb wird auf die Angabe der Metalldispersion bei diesen Katalysatoren verzichtet, da diese nur eine begrenzten Aussagekraft enthält. | | | |

Bei dem Nitrobenzolniederdrucktest gelten die folgenden Reaktionsparameter:

| | |
|---|---|
| Reaktionsdruck | 10 mbar |
| Temperatur | 30°C |
| Lösungsmittel | Isopropanol/Wasser = 4:1 |
| Rührerdrehzahl | 2000 UpM |

Es werden jeweils 10 ml Nitrobenzol mit 200 mg Katalysator in 150 ml Lösungsmittel in eine Wasserstoff gespülte Glasapparatur gegeben. Die Reaktion und damit die Wasserstoffaufnahme wird durch Anstellen des Rühres gestartet. Nach einer 3 minütigen Vorlaufzeit wird die Aktivität in der Einheit [ml H₂/min. •g] mittels Massendurchflussmesser während 5 Minuten gemessen.

### Anwendungsbeispiele

Die Katalysatoren gemäß den Beispielen 1 bis 8 werden hinsichtlich ihrer Aktivität und Selektivität in der diskontinuierlich durchgeführten Hydrierung von Dinitrotoluol zur Toluoldiamin getestet. Dabei werden die folgenden Reaktionsparameter eingehalten:

| | |
|---|---|
| Reaktionsdruck | 10 bar |
| Temperatur | 100°C /120°C |
| Lösungsmittel | Toluoldiamin/Wasser |

Es werden jeweils 40 g Dinitrotoluol, gelöst in 160 g Toluoldiamin/> 36% Wasser, quantitativ hydriert. Der Endpunkt der Reaktion ist durch den rapiden Abfall der Wasserstoffaufnahme auf Null präzise bestimmbar. Es wird stets eine Katalysatormenge von 0,5 Gew.-%, bezogen auf eingesetztes Dinitrotoluol, verwendet. Die Nebenprodukte in dem Reaktionsprodukt werden anschließend mit GC (Gaschromatographie) bestimmt.

Die entstandenen Nebenprodukte werden in drei Gruppen eingeteilt:
- Nebenprodukt 1:: Ringhydrierte Folgeprodukte des Dinitrotoluols, Methylnitroanilins und Toluoldiamins
- Nebenprodukt 2:: Unvollständig hydrierte Verbindungen (zum Beispiel Methylnitroanilin)
- Nebenprodukt 3:: Dimere, Oligomere ("Teerbildung")

Die Proben werden nach einer Rührzeit von 15 Minuten nach dem Ende der Wasserstoffaufnahme aus dem Autoklaven entnommen.
Die Ergebnisse der Hydrierung bei 10 bar sind in der folgenden Tabelle zusammengefasst.

| **Katalysator nach Beispiel** | **Hydrierzeit [min.]** | **Ausbeute TDA [Gew%]** | **Nebenprodukt** | | |
|---|---|---|---|---|---|
| | | | 1 [Gew%] | 2 [Gew%] | 3 [Gew%] |
| **1** | 27 | 97,8 | 0,8 | / | 1,4 |
| **2** | 30 | 98,1 | 0,8 | 0,8 | 0,3 |
| **3** | 35 | 92,5 | 2,2 | 1,9 | 3,4 |
| **4** | 26 | 94,4 | / | / | 5,6 |
| **5** | 25 | 98,8 | / | / | 1,2 |
| **6** | 40 | 83,2 | 4,2 | 1,2 | 11,4 |
| **7** | 35 | 98,8 | / | 0,7 | 0,6 |
| **8** | 32 | 99,6 | / | 0,1 | 0,3 |

## Patentansprüche

1. Hydrierkatalysator, enthaltend als Kohlenstoffträger einen Ruß mit einem H- Gehalt von > 4000 ppm, und als katalytisch aktive Komponente Palladium und/oder Platin oder bi- und/oder multimetallisch dotiertes oder legiertes Palladium und/oder Platin.

2. Hydrierkatalysator nach Anspruch 1, **dadurch gekennzeichnet, daß** das Palladium und/oder Platin mit Elementen aus der Reihe Ru, Rh, Fe, V, Sn oder Kombinationen davon dotiert beziehungsweise legiert ist.

3. Hydrierkatalysator nach Anspruch 2, **dadurch gekennzeichnet, daß** das Atomverhältnis zwischen Palladium und/oder Platin und gegebenenfalls den Dotierungs- beziehungsweise Legierungskomponenten zwischen 200:1 und 1:200 ist.

4. Hydrierkatalysator nach Anspruch 1, **dadurch gekennzeichnet, daß** Palladium und/oder Platin in einer Menge zwischen 0,05 und 80 Gew.-%, bezogen auf das Gesamtgewicht des Katalysators, vorliegt.

5. Verfahren zur Herstellung eines Hydrierkatalysators nach Anspruch 1, **dadurch gekennzeichnet, daß** zu einer Suspension eines Rußes mit einem H-Gehalt >4000 ppm, Edelmetallsatzlösung und gegebenenfalls Salzlösungen der Dotierungs- beziehungsweise Legierungselemente gleichzeitig, nacheinander oder in einem zweistufigen Verfahren nach vorheriger Herstellung eines Edelmetallvorkatalysators zugegeben wird, durch Verwendung einer basischen Verbindung die (Edel-) Metallsalzlösungen hydrolysiert werden und die vollständige Abscheidung des Edelmetalls beziehungsweise der anderen Metalle durch Reduktion mit einem Reduktionsmittel durchgeführt wird.

6. Verfahren zur Herstellung eines Hydrierkatalysators nach Anspruch 1, **dadurch gekennzeichnet, daß** nach naßchemischer Präparation des Hydrierkatalysators eine Temperaturbehandlung unter Inertgas oder reduzierender Atmosphäre bei Temperaturen zwischen 0°C und 1000°C durchgeführt wird.

7. Verwendung des Katalysators nach Anspruch 1 zur Hydrierung von Nitrobenzol zu Anilin und Dinitrotoluol zu Toluoldiamin.

8. Verfahren zur Herstellung von Anilin und Toluoldiamin, **dadurch gekennzeichnet, daß** die katalytische Hydrierung der korrespondierenden Nitroverbindung in Flüssigphase als kontinuierlich oder diskontinuierlich betriebener Prozess, bei Drücken zwischen 1 und 100 bar und Temperaturen zwischen 0°C und 250°C, in Gegenwart des Katalysators nach Anspruch 1 durchgeführt wird.
